# EUROPEAN PATENT SPECIFICATION

(11) **EP 4 298 915 B1**
(45) Date of publication and mention of the grant of the patent: **04.06.2025**
(21) Application number: 22181698.6
(22) Date of filing: 28.06.2022
(51) Int. Cl.: A23G 1/00, A23G 1/18, G01N 25/04, G01N 33/02

(54) **QUALITY CONTROL OF CONFECTIONERY MASS**
QUALITÄTSKONTROLLE VON SÜSSWARENMASSE
CONTRÔLE QUALITÉ D'UNE PÂTE DE CONFISERIE

(43) Date of publication of application: 03.01.2024
(73) Proprietor: Aasted ApS, 3520 Farum (DK)
(72) Inventor: Holmud, Dennis, 2860 Søborg (DK)
(74) Representative: Heiden, Finn

(56) References cited:
- EP-A1- 2 941 960
- EP-A1- 3 685 675
- EP-A2- 2 510 806
- HASLUND HENNING: "Chocolate Tempering Controlling tempering through the production process minimizes the risk of faulty manufacture.", THE MANUFACTURING CONFECTIONER, 1 September 2010 (2010-09-01), pages 95 - 102, XP055902367

## Description

The present invention concerns a method, according to claim 1, for testing and continuous tempering of a
liquid, fat-containing, crystallizable confectionery mass, whereby a sample of the mass is extracted from the liquid mass before being tempered, further being cooled, crystallized and solidified, simultaneously providing a temper curve.

Testing methods are widely known in the food industry whenever confectionery mass is to be tested before or during production. The temper curve is evaluated as the curve shape is regarded as characterizing the "quality" of the obtainable or achieved temper of the particular mass. Especially, the inclination or slope of a line through the point of inflection of the curve is regarded as a strong and reliable indicator for the tempering qualities of the particular mass. The slope of the line is often graduated as a tempering index having a range of 0-10.

It is well known that it is a challenge to prepare and achieve high quality confectionery mass, such as for example chocolate mass, each time a recipe is altered. It could be by addition of other emulsifiers or types of fat, or when the tempering procedure itself is amended in temperatures or in time. Though the temper index measured is equal at a subsequent production, it does not mean at all, that the same crisp break, long shelf life or shiny appearance will be obtained.

The masses applied within the invention are any confectionery mass having a fat-content, that is able to crystallize into a stable, solid mass. It could for example be chocolate or chocolate-like mass, which may encompass all types of suspensions of non-fat particles such as sugar, milk powders and cocoa solids mixed up with a liquid fat constituent, so that the suspensions are capable of crystallizing. It could be chocolate or chocolate-like mass types used in any kind of production of confectionery articles.

When it comes to the most widely used chocolate mass types, the fat constituent comprises genuine cocoa butter typically in a content of up to approximately 35%. However, the fat phase may also comprise substitutes as well. A small content of up to 2-3 % of genuine cocoa butter may still be left in the recipe. Substitutes may be in the form of other types of fat-containing oils such as palm-kernel oil. Chocolate types having the cocoa butter been replaced by other fats are often named commercially as compound chocolate, especially when the cocoa butter has been replaced completely by palm-kernel oil.

For the continuous tempering of the confectionery mass to be performed, it is decisive, that whether the fat phase constitutes of genuine cocoa butter or substitutes, the fat phase must be capable of crystallizing into stable crystal types, such as the βV-crystals developing in genuine cocoa butter. However, it is important to avoid instable crystals in the solidified mass. Only then, eatable chocolate articles with good taste, crisp break and shiny appearance are created. The solidified chocolate articles will also achieve the longest possible shelf life and the best resistance against bloom, as instable crystals are diminished. If there is a content of in-stable crystals left in the mass, they will give rise to shorter shelf-life as the articles will bloom more quickly as when in-stable crystals are not present.

For the manufacturers of articles made by such masses, it is always desirable, that the tempering process and apparatus can be adjusted to deliver tempered mass having a content of the stable crystal-type only, such as βV-crystals in chocolate mass. Only then, the manufacturer can rely on, that the quality of their chocolate products is consistent. Consequently, the manufacturer is testing their mass before and during the tempering by means of a temper curve and evaluating the shape and temper index thereof.

In an example useful for understanding the invention, a tempermeter is disclosed, the tempermeter comprising a casing having a cooling unit at the top carrying a cup with the sample as well as a computer unit adapted to calculate and provide a temper curve. The temper curve is printed on a strip chart recorder or shown on a screen or both. The computer is adapted to calculate the point of inflection and mark it at the temper curve. The slope of the line at the point of inflection may also be calculated and displayed as well as the temper index may.

The invention also relates to a production line according to 3 comprising, *inter alia,* at least a tempering apparatus, which has an in-line tempermeter.

The in-line tempermeter has a unit arranged at the continuous mass flow conduit and is adapted to extract a sample of the mass. The tempermeter has a cooling unit adapted to cool the sample, and is connected to a computer unit adapted to calculate and provide a temper curve and calculate the size of the area above or below the temper curve and a control means for the cooling circuit of the tempering apparatus is connected with the computer unit, which is further adapted to convert the actual size of the area to an expression for the actual energy amount required to cool and solidify the particular mass sample.

The invention also relates to a computer program according to claims 5-8, that in preferred embodiments provides a temper curve for the above mentioned apparatuses, as well as calculates a point of inflection on the curve.

Haslund, Henning: "Chocolate Tempering Controlling tempering through the production process minimizes the risk of faulty manufacture", The Manufacture Confectioner, 1 September 2010 (2010-09-01), pages 95-102, XP055902367 discloses a survey over different testing methods available, as well as factors and measures to take into account when tempering chocolate mass during production for obtaining a fine and attractive result of the solidified chocolate articles. Focus is on obtaining a suitable number of crystals seeds and especially the attractive Beta V crystals in the tempered chocolate mass. By performing the tempermeter test differently obtained tempering curves are discussed; "overtempered, well-tempered and under-tempered," and the impact on the constitution of the particular tempered chocolate mass. In practical use it is recommended, that the so-called Temper Index is calculated for the particular tempering curve obtained, and used as a concrete numerical value for recording and communication. When discussing the challenge of how the tempering machine should be set in practice to obtain the correct degree of tempering, the conclusion is, that it can only be found by trials. At each trial the tempermeter test must be performed and the tempercurves evaluated until they are satisfactory, whereafter that the production of tempering the mass with the particular settings can begin.

EP2941960A1, Aasted ApS discloses a tempering apparatus having a cooling stage A, a crystallisation stage B and a mixing stage C. A CPU is connected with temperature sensors at the output of cooled mass from stage A, at the output of mass from the cooling stage B and from the output of the tempered chocolate mass from the mixing stage C. A part of the mass steam leaving stage A is by-passed stage B and supplied to stage C. Premature tempering tests are carried out for the particular mass and the temper curves are obtained until the desired tempering degree, temper index value and output temperature of the tempered mass are obtained and recorded. The values are coded into the CPU as well as an equation. When tempering is started the operator gives in the desired values to be constant; the output temperature and the temper index value. The CPU then controls the tempering process automatically.

EP2510806A2, Sollich KG discloses a tempering apparatus with a programmable logic controller and an in-line tempermeter arranged after the cooling and crystallisation stages. With regular time intervals mass probes are obtained by the in-line tempermeter and results of tempercurves and temper degree, such as temper index value, are maintained by the software of the logic controller. The software evaluates if the temperature of the tempered mass is constant and if the desired temper degree is constant, and if not confirmative, the software compares with the values obtained by preceeding results. The software then calculates an incremental regulation of the process controlling parameters such as the cooling water temperature or flow of the stages. The evaluation procedure goes on and on automatically.

EP3685675A1, Aasted ApS discloses a tempering apparatus controlled by a HMI-unit with a touch-screen. A set-point value for the outlet temperature of the tempered mass is entered at the touch screen and the crystallisation amount to be achieved is adjusted as a control value on a scale at the touch-screen. The HMI-unit calculates and transmits a value for the water temperature cooling of the crystallisation stage whereafter the HMI-unit continuedly repeats; receiving the actually measured outlet temperature, calculating deviation from the set-point value, if any, calculating and transmitting a new set-point value for the water temperature cooling. The simplicity of the few numbers to be coded in, and the automatic control during tempering, makes the tempering apparatus straightforward to operate.

It is well-known, that though the measured temper index is approximately equal for different batches of the same mass recipes, difficulties in relation to control and renewed setting of process parameters of the tempering and cooling of the masses in the production is often experienced.

A severe challenge in the production of articles using prior art temper curve methods are, that the measured temper index is approximately equal regardless of the setting of the cooling temperature of the mass being tempered. For many mass types it means, that even if the cooling temperature is several degrees higher than the optimal cooling temperature for crystallization of that mass, the observed temper index is approximately the same.

So, if the operator is not observant to the cooling water temperature read-out, there is a severe risk, that the production is proceeding to the end with a to high cooling temperature, i.e. such as for example 3-5 degrees higher than the optimal cooling temperature for the particular mass crystallization. The result is, that the mass is not sufficiently crystallized and that the whole batch of articles made is waisted. The required tempering quality is not achieved. Gloss, crisp break, taste and shelf life are not perfect.

The problem to be solved by the present invention is to provide a method for testing and continuous tempering of a liquid, fat-containing, and crystallizable confectionery mass, by which is obtained values, that represent trustable characteristics for the particular mass.

The method according to the invention is defined in claim 1.

Hereby is obtained that the area values are directly appliable when comparing different batches of mass and are directly transferable to the setting and control of the production line control parameters, such as in the cooling control of the tempering apparatus.

As the sample is extracted from the liquid mass before being tempered, the size of the area calculated is a genuine expression for the heating amount needed to be removed from the liquid, crystal free mass sample. The new method then provides the manufacturer with an unforeseen reliable result in testing different batches of mass before tempering, achieving fully comparable and trustable values.

According to the method of the invention, samples are extracted continuously from the tempered mass during continuous tempering in a production line. The actual size of the area above or below the temper curve is continuously calculated by the computer, and the tempering process is regulated so that the size of the area is maintained at a desired, constant level. Consequently, the manufacturer is sure, that the produced mass articles are of the high tempering quality they choose, even as the production runs automatically.

It is further advantageous, when the cooling water temperature for the mass being tempered is lowered, then the actual size of the area is increased, and when the cooling water temperature for the mass being tempered is raised, then the actual size of the area is decreased.

With the inventive production line comprising an in-line tempermeter, the computer unit is further adapted to calculate the size of the area below or above the temper curve, and further convert the actual size of the area to an expression for the actual energy amount required to cool and solidify the particular sample and mass in a production.

The computer unit is connected with the control means for the cooling circuit of a tempering apparatus. The computer unit may also be connected with the control means for a cooling unit of a cooling tunnel in a production line.

According to a preferred embodiment of the invention, the computer program comprises the instructions, that it determines a starting point of a maximum temperature of the temper curve, it determines a first point of inflection of the temper curve, it determines at least one further second point of inflection of the temper curve, and that it is calculating the area above the temper curve limited by:
the temper curve, a horizontal upper line through the starting point and a vertical line through the second point, or is calculating the area below the temper curve as limited by:
a vertical line through the starting point and a horizontal lower line through the second point.

Hereby is obtained a very simple manner of achieving a highly accurate result and expression representing the actual energy amount required to cool, crystallize and solidify the particular mass sample. The result in the form of a size of the area is directly comparable with results from earlier or subsequent mass sample test according to the invention. The results are easily comparable on a screen to the non-skilled operator of a line.

The computer program may advantageously convert the area size to an actual energy amount simply by dividing with a factor in the range of 1-1000, advantageously 1-100.

An even further accuracy in the calculation of the area size is obtained when the computer program instead of the second point of inflection uses a third point of inflection limiting the area in the calculations. The calculations are then done by the computer by means of simple integral mathematics adapted to the temper curve in question.

The invention is explained further below under reference to preferred embodiments as well as the drawing, in which
fig. 1 is a schematic view of a tempermeter apparatus comprising a computer unit. The tempermeter *per se* is outside the subject-matter of the claims.
fig. 2 is a picture of a temper curve provided with the tempermeter of figure 1 and printed on strip chart recorder together with the process parameters achieved,
fig. 3 is a picture of a further temper curve provided with the same chocolate mass and the tempermeter of fig. 2, however, with different process settings recorded,
fig. 4 is a picture of an even further temper curve provided with the tempermeter 1 of figure 1, comprising a further, highly accurate example, useful for understanding the invention, of a computer program calculating the area above the temper curve,
fig. 5 is a picture of the same temper curve as disclosed in figure 4, however with the computer program calculating the area below the temper curve,
fig. 6 is a schematic view of a production line apparatus according to the invention, comprising a tempering apparatus with an in-line tempermeter as well as a computer unit and program adapted to execute the steps according to the invention, seen from the side,
fig. 7 is a schematic side view of the in-line tempermeter unit, and
fig. 8 is a picture of a screen dump from the tempering machine disclosing process parameters and a temper curve.

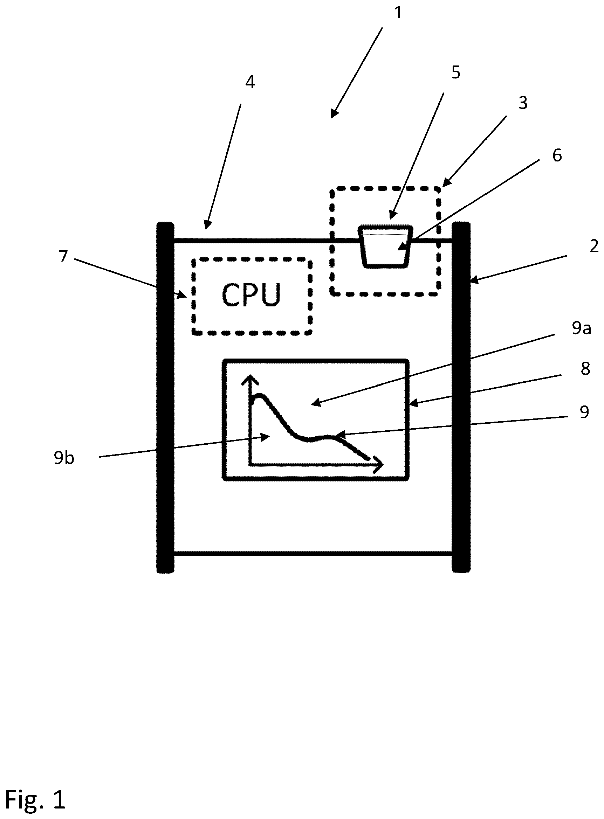

The tempermeter apparatus 1 disclosed in figure 1 has a casing 2 and a schematically depicted cooling unit 3 at the top 4. The cooling unit 3 is carrying a metal cup 5 with a sample 6 of confectionery mass, such as of chocolate mass. The mass sample 6 is typically extracted from a major batch of confectionery mass before it is brought into the production. The mass sample may also be taken out from the mass flow in a production line, for example after it has been tempered and before it is deposited into articles.

The mass sample 6 is cooled, crystallized and solidified in the metal cup 5 by the cooling unit 3. Simultaneously is provided a temper curve 9 at the touch screen 8 of the tempermeter 1.

The computer unit 7 is adapted to calculate and provide the temper curve 9 as with the prior art tempermeters. However, in addition, the tempermeter 1 is also adapted to calculate the size of the area above 9a or below 9b the temper curve 9.

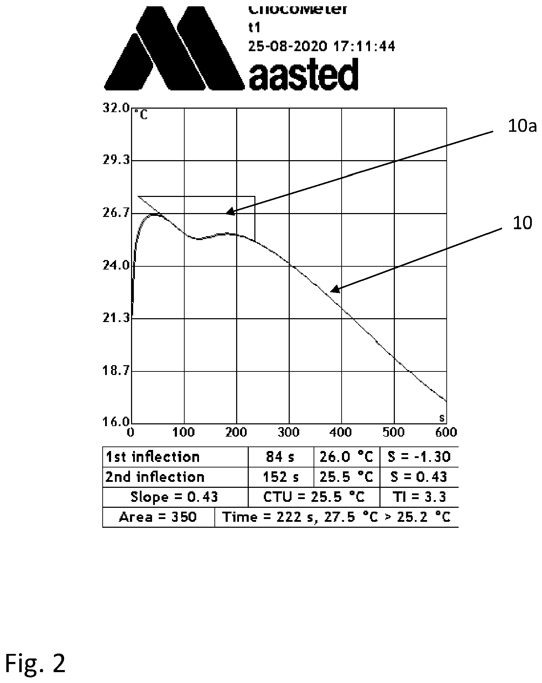

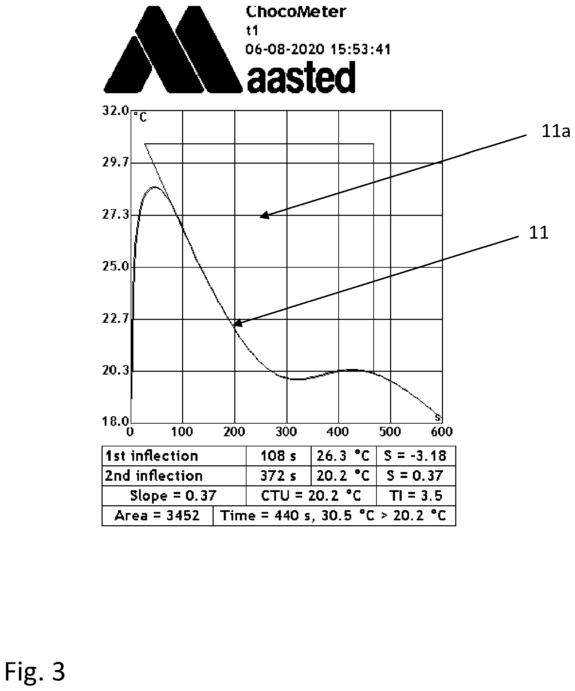

In figures 2 and 3 are disclosed two different temper curves 10, 11 obtained with samples from the same dark chocolate batch, however, undergoing different cooling treatments through a tempering apparatus.

The dark chocolate of figure 2 is correctly tempered as indicated by the temperature being 27.5 °C when leaving the tempering apparatus. The slope in the 2. point of inflection is 0.43 and consequently the temper index is calculated to be 3.3. Every calculation is depicted at the bottom of the read out of figures 2 and 3.

Referring to the dark chocolate sample of figure 3, the slope in 2. point of inflection is 0.37 and the temper index is then 3.5. The temperature being 30.5 °C when leav-
ing the tempering apparatus - it actually means, that the temperature is 3.0 °C too high in comparison to the well-tempered mass of figure 2. as the mass has not been sufficiently cooled and tempered.

However, this fact is not being revealed by a simple comparison of the temper index of 3.5 of the under-tempered batch of figure 3 with the temper index of 3.3 of the well-tempered mass of figure 2.

So, when contemplating the temper index only, as traditionally done, the operator gets no hints as far as to the fact that the mass could be severely far from being properly tempered, i.e. such as not being cooled sufficiently and consequently being under-tempered as in figure 3.

The area 10a above the temper curve 10 of figure 2 is calculated by the computer CPU 7 of figure 1 by applying traditional integral mathematics for curves. The calculated area 10a is 350mm2. The area 11a above the temper curve 11 of figure 3 is calculated by the computer 7 to be 3452mm2.

However, when comparing the area of 350mm2 of fig. 2 with the area of 3450mm2 of fig. 3 it is pretty evident, that it is around 10 times bigger. Then, the operator is in no doubt of that the two masses are differently tempered.

Applying the actual size of the area calculated above or below the temper curve as representing the size of the actual energy amount required to cool, crystallize and solidify the particular mass, as required by the method according to the invention, surely reveals to the operator in a simple manner, that they must amend the settings of the cooling energy to achieve a properly tempered mass and consequently a high-quality chocolate production.

The above example of figure 3 reveals to the operator, that they must increase the cooling energy so that the area 11a above the temper curve 11 is reduced to the size of the area 10a in figure 10 of the well-tempered mass before they can start the production of the mass articles.

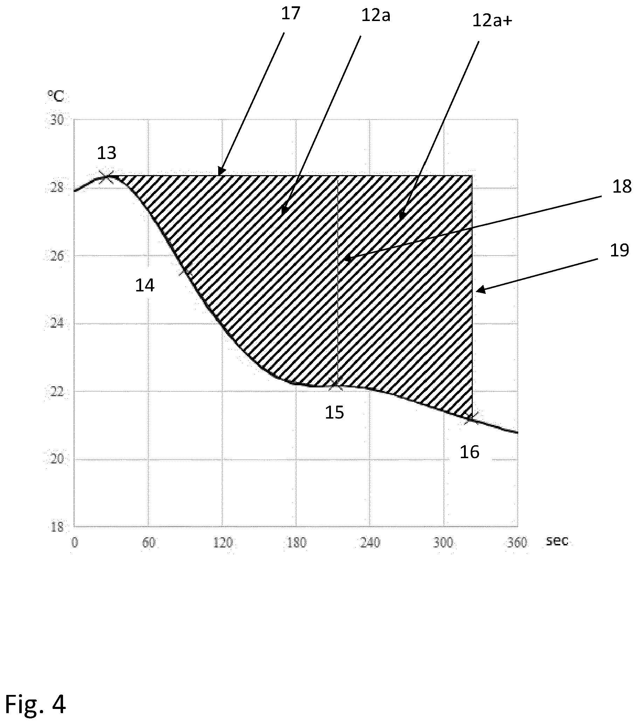

In figure 4 is disclosed a temper curve of a sample of a milk chocolate provided with the tempermeter 1 of figure 1, comprising a further, highly accurate example, useful for understanding the invention, of a computer program calculating the area 12a above the temper curve 12.

The program determines a starting point 13 of a maximum temperature of the temper curve 12. It determines a first point of inflection 14, and a second point of inflection 15. When the highest accuracy is to be obtained a third point of inflection 16 is determined as well.

The computer program then calculates the area 12a limited by the temper curve 12, a horizontal line 17 through the starting point 13 and a vertical line 18 through the second point 16. When the highest accuracy is to be obtained the computer program calculates the area 12a+ limited by the temper curve 12, the horizontal line 17 through the starting point 13 and a vertical line 19 through the third point of inflection 16.

Instead of calculating the area above the temper curve 12, the area below the temper curve 12 may be calculated within the inventive idea.

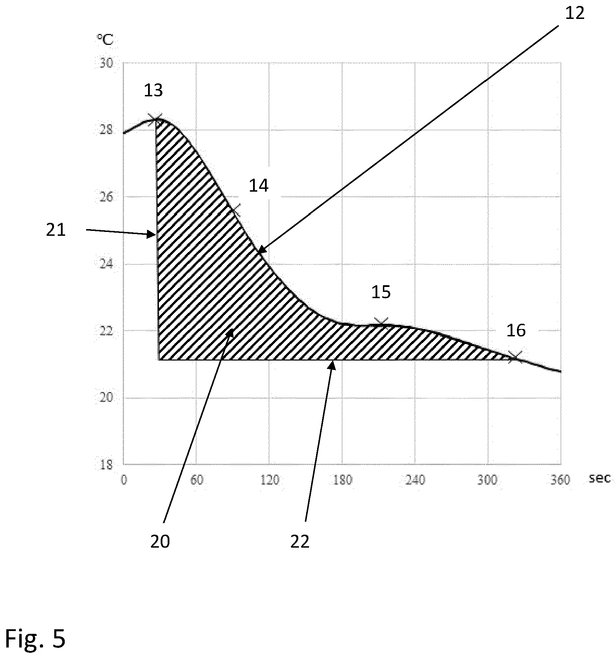

In figure 5 is disclosed the same temper curve 12 as in figure 4. The starting point 13 of the maximum temperature, the first point of inflection 14, the second point of inflection 15 and the third point of inflection 16 are all calculated by the computer program and marked at the temper curve as in figure 5. However, different is, that the area 20 below the temper curve 12 is calculated as limited by the temper curve 12, a vertical line 21 through the starting point 13 and a horizontal line 22 through the third point 16.

The production line apparatus 23 according to the invention, is comprising a tempering apparatus 24 with an in-line tempermeter 25 as well as a computer unit CPU 26 and program adapted to execute the steps according to the invention.

The in-line tempermeter is arranged in the mass flow conduit 27, which connects the temperer 24 with a depositor 28, depositing the liquid, tempered chocolate into underlying moulds 29. The moulds 29 are arranged on a belt or is engaging with a chain drive 30 transporting the moulds through the cooling tunnel 31.

The computer 26 of the tempering apparatus 24 is connected via wires 32 with the in-line tempermeter 25, so that the results and temper curves 33 provided by the in-line tempermeter 25 is displayed on the touchscreen 34 at the front of the tempering apparatus 24.

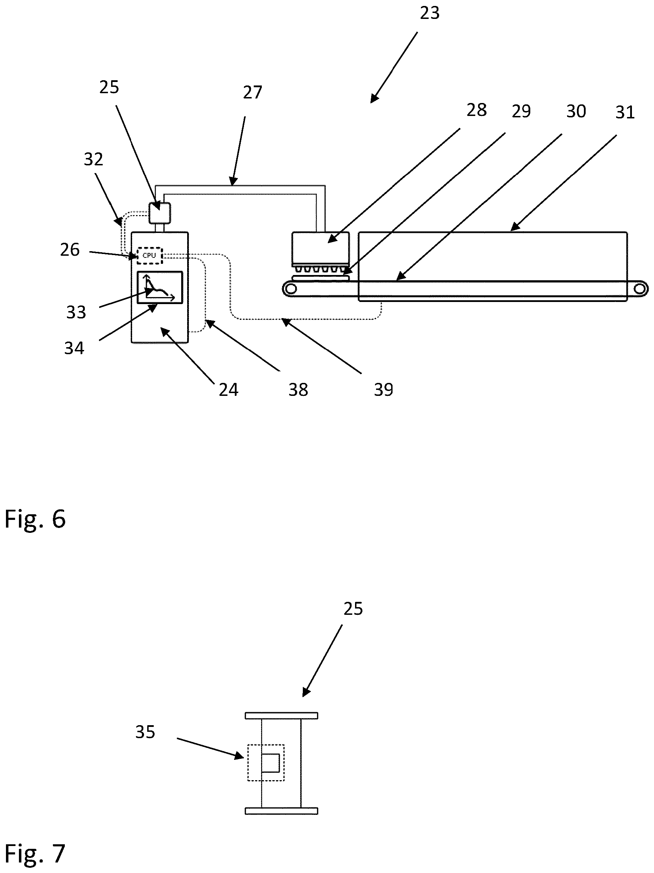

The in-line tempermeter 25 itself is schematically disclosed in figure 7. It has a cooling unit 35 in which is arranged a known device for collecting a sample of mass either in the mass flow or for extracting the sample therefrom and cool it, simultaneously providing a temper curve 33 at the screen 34 of the tempering apparatus 24.

During continuous tempering of the mass and solidification of the mass articles the cooling tunnel 31 in the production line 23, samples are provided continuously from the tempered mass with the in-line tempermeter 25. The actual size of the area above or below the temper curve 33 is maintained at a desired, constant level.

The size of the area could be calculated by the computer 26 as explained above under reference to either of figures 2-5.

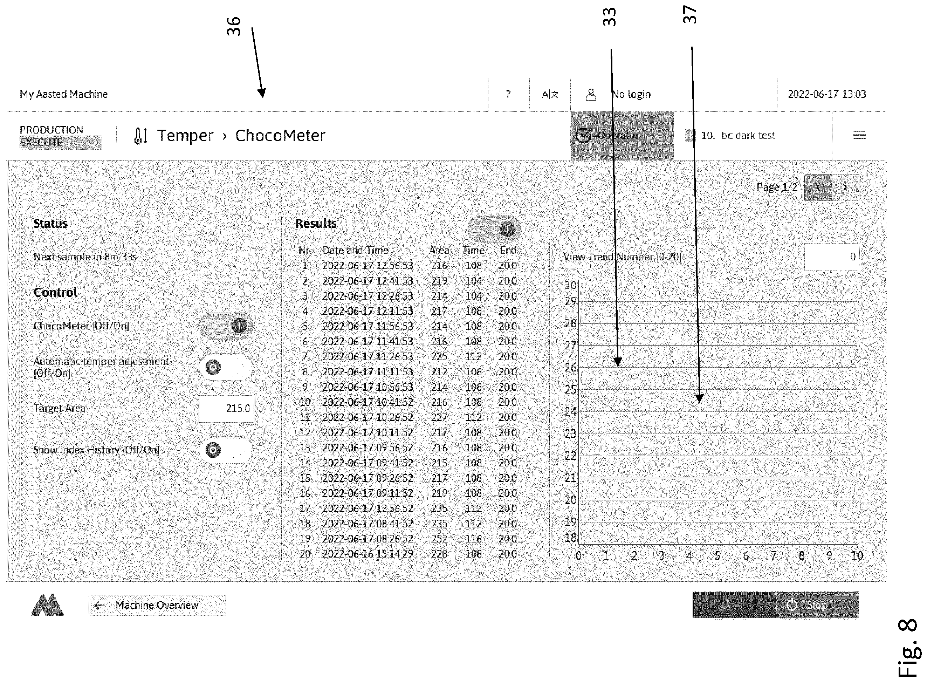

In figure 8 is disclosed a screen dump 36 from the screen 26 of the tempering apparatus 24 during a production run with dark chocolate. The computer 26 calculates continuously the size of the area 38 above the temper curve 33 as explained in relation to figure 4, i.e. calculating the best mode of the area 12a+ above the temper curve 12.

Before starting the production run, premature tests have disclosed, that a target area of 215 mm2 calculated above the temper curve 33 provides the most well-tempered quality for the particular chocolate mass. The size of 215.0 mm2 of the target area 37 during production run is then coded into the computer 26 via the touchscreen 34, as disclosed to the left side of the screen dump 36.

At the column "Results" of the screen dump 36 is disclosed data for each of the continuously taken samples by the in-line tempermeter 25. Date and time as well as computer calculated areas above the temper curves for the particular sample taken.

Wires 38 connect the computer 26 with non-disclosed control means for the cooling circuit of the tempering apparatus. The computer 26 then regulates the cooling energy in accordance with the size of the target area. In the present example the cooling water temperature for the mass being tempered is lowered when the actual size of the area is increased in relation to the target area, and the cooling water temperature for the mass being tempered is raised when the actual size of the area is decreased in relation to the target area.

The control of the tempering machine is then performed automatically without any knowledge of the actual temper index.

Wires 39 connect the computer 26 with non-disclosed control means for the cooling circuit of the cooling tunnel 31. The computer 26 then regulates the cooling energy of the cooling tunnel 31 in accordance with the size of the target area and likewise as with the tempering apparatus 24 as described above.
- 1:: tempermeter apparatus
- 2:: casing
- 3:: cooling unit
- 4:: top
- 5:: cup
- 6:: mass sample
- 7:: computer unit CPU
- 8:: touch screen
- 9:: temper curve
- 9a:: area above temper curve
- 9b:: area below temper curve
- 10:: temper curve
- 10a:: area above temper curve
- 11:: temper curve
- 11a:: area above temper curve
- 12:: temper curve
- 12a:: area above temper curve
- 12a+:: area above temper curve
- 13:: starting point of maximum temperature
- 14:: first point of inflection
- 15:: second point of inflection
- 16:: third point of inflection
- 17:: horizontal line
- 18:: vertical line
- 19:: vertical line
- 20:: area below temper curve
- 21:: vertical line
- 22:: horizontal line
- 23:: production line apparatus
- 24:: tempering apparatus
- 25:: in-line tempermeter
- 26:: computer unit
- 27:: conduit
- 28:: depositor
- 29:: moulds
- 30:: chain drive
- 31:: cooling tunnel
- 32:: wires
- 33:: temper curves
- 34:: touch-screen
- 35:: cooling unit
- 36:: screen dump
- 37:: area above temper curve
- 38:: wire
- 39:: wire

## Claims

1. Method for testing and continuous tempering of a liquid, fat-containing, crystallizable confectionery mass, wherein a sample (6) of the mass is extracted from the liquid mass before being tempered , further being cooled, crystallized and solidified, simultaneously providing a temper curve (9, 10, 12, 33), **characterized in that** at least part of the size of the area above (9a, 10a, 11a, 12a, 12a+, 37) or below (9b, 20) the temper curve (9, 10, 11, 12, 33) is calculated, and **in that** the actual size of the area calculated is applied as representing the actual energy amount required to cool, crystallize and solidify the particular mass sample (6), that the actual size of the area calculated is applied in further production of mass articles as representing the actual energy amount required to cool, crystallize and solidify the particular mass, and **in that** during continuous tempering and solidification of the mass in a production line (23), samples are provided continuously from the tempered mass as the actual size of the area above (37) or below the temper curve (33) is continuously calculated by a computer and the tempering process is regulated so that the size of the area is maintained at a desired, constant level.

2. Method according to claim 2, **characterized in that** the cooling water temperature for the mass being tempered is lowered when the actual size of the area (37) is increased, and the cooling water temperature for the mass being tempered is raised when the actual size of the area (37) is decreased.

3. Production line (23) having means for controlling continuous tempering of a fat-containing, crystallizable confectionery mass and execute the steps of one or more of claims 1-2, which production line (23) comprises at least a tempering apparatus (24), which tempering apparatus (24) comprises an in-line tempermeter (25) with a unit arranged at a continuous mass flow conduit (27), which unit is adapted to provide a sample of the mass, further having a cooling unit (35) adapted to cool the mass sample, and a computer unit (26) connected (32) with the in-line tempermeter and adapted to calculate and provide a temper curve (33) and calculate the size of the area above (37) or below the temper curve (33) and further adapted to convert the actual size of the area to an expression for the actual energy amount required to cool and solidify the particular mass sample, and wherein a control means for the cooling circuit of the tempering apparatus (24) is connected with the computer unit (26).

4. Production line according to claim 3, further comprising a cooling tunnel (31) for the confectionery articles, that the control means for the cooling unit of the cooling tunnel (31) is connected (39) with the computer unit (26).

5. A computer program comprising instructions to cause the computer-unit of one or more of claims 3 or 4 to execute one or more of the steps of claims 1 or 2.

6. Computer program according to claim 5 comprising the instructions:
determining a starting point (13) of a maximum temperature of the temper curve (12),
determining a first point (14) of inflection of the temper curve (12),
determining at least one further second point (15) of inflection of the temper curve (12),
calculating the area (12a) above the temper curve (12) limited by:
the temper curve (12), a horizontal upper line (17) through the starting point (13) and a vertical line (18) through the second point (15), or
calculating the area (20) below the temper curve (12) as limited by:
a vertical line (21) through the starting point (13) and a horizontal lower line (22) through the second point (15).

7. Computer program according to claim 5 or 6 comprising the instructions:
determining a starting point (13) of a maximum temperature of the temper curve (12),
determining a first point (14) of inflection of the temper curve (12),
determining second (15) and a third (16) points of inflection of the temper curve (12),
calculating the area above (12a+) the temper curve (12) limited by:
the temper curve (12), a horizontal upper line (17) through the starting point (13) and a vertical line (19) through the third point (16), or
calculating the area (20) below the temper curve (12) limited by:
a vertical line (21) through the starting point (13) and a horizontal lower line (22) through the third point (16).

8. Computer program according to one or more of claims 5-7 comprising the instructions that,
the calculated size of the area above (12a, 12a+) or below (20) the temper curve (12) is divided by a factor in the range of 1-1000, advantageously 1-100, thereby converting it to an actual energy amount required to cool and solidify the particular sample.

## Patentansprüche

1. Verfahren zum Prüfen und kontinuierlichen Tempern einer flüssigen, fetthaltigen, kristallisierbaren Süßwarenmasse,
wobei eine Probe (6) der Masse aus der flüssigen Masse extrahiert wird, bevor sie getempert wird, weiter gekühlt, kristallisiert und verfestigt wird, wobei gleichzeitig eine Temperkurve (9, 10, 12, 33) bereitgestellt wird,
**dadurch gekennzeichnet, dass** mindestens ein Teil der Größe der Fläche oberhalb (9a, 10a, 11a, 12a, 12a+, 37) oder unterhalb (9b, 20) der Temperkurve (9, 10, 11, 12, 33) berechnet wird, und dass die tatsächliche Größe der berechneten Fläche als die tatsächliche Energiemenge repräsentierend angewendet wird, die zum Kühlen, Kristallisieren und Verfestigen der jeweiligen Massenprobe (6) erforderlich ist, dass die tatsächliche Größe der berechneten Fläche bei der weiteren Produktion von Massenartikeln als die tatsächliche Energiemenge repräsentierend angewendet wird, die zum Kühlen, Kristallisieren und Verfestigen der jeweiligen Masse erforderlich ist,
und dass während des kontinuierlichen Temperns und Verfestigens der Masse in einer Produktionslinie (23) kontinuierlich Proben aus der getemperten Masse bereitgestellt werden, da die tatsächliche Größe der Fläche oberhalb (37) oder unterhalb der Temperkurve (33) kontinuierlich computergestützt berechnet wird und der Temperprozess so geregelt wird, dass die Größe der Fläche auf einem gewünschten, konstanten Niveau gehalten wird.

2. Verfahren nach Anspruch 2, **dadurch gekennzeichnet, dass** die Kühlwassertemperatur für die zu tempernde Masse bei Vergrößerung der tatsächlichen Größe der Fläche (37) abgesenkt wird, und die Kühlwassertemperatur für die zu tempernde Masse bei Verkleinerung der tatsächlichen Größe der Fläche (37) erhöht wird.

3. Produktionslinie (23) mit Mitteln zum Steuern des kontinuierlichen Temperns einer fetthaltigen, kristallisierbaren Süßwarenmasse und zum Durchführen der Schritte nach einem oder mehreren der Ansprüche 1-2, wobei die Produktionslinie (23) mindestens eine Tempervorrichtung (24) umfasst, wobei die Tempervorrichtung (24) ein Inline-Tempermeter (25) mit einer an einer kontinuierlichen Massenflussleitung (27) angeordneten Einheit umfasst, wobei die Einheit eingerichtet ist, um eine Probe der Masse bereitzustellen, ferner mit einer Kühleinheit (35), die eingerichtet ist, um die Massenprobe zu kühlen, sowie einer Rechnereinheit (26), die mit dem Inline-Tempermeter verbunden (32) ist und eingerichtet ist, um eine Temperkurve (33) zu berechnen und bereitzustellen und die Größe der Fläche oberhalb (37) oder unterhalb der Temperkurve (33) zu berechnen, und ferner eingerichtet ist, um die tatsächliche Größe der Fläche in einen Ausdruck der tatsächlichen Energiemenge zu konvertieren, die zum Kühlen und Verfestigen der jeweiligen Massenprobe erforderlich ist, und wobei ein Steuermittel für den Kühlkreislauf der Tempervorrichtung (24) mit der Rechnereinheit (26) verbunden ist.

4. Produktionslinie nach Anspruch 3, ferner umfassend einen Kühltunnel (31) für die Süßwarenartikel, dass das Steuermittel für die Kühleinheit des Kühltunnels (31) mit der Rechnereinheit (26) verbunden (39) ist.

5. Computerprogramm, das Anweisungen umfasst, um zu bewirken, dass die Rechnereinheit nach einem oder mehreren der Ansprüche 3 oder 4 einen oder mehrere der Schritte nach Anspruch 1 oder 2 ausführt.

6. Computerprogramm nach Anspruch 5, umfassend die Anweisungen:
Bestimmen eines Startpunkts (13) einer maximalen Temperatur der Temperkurve (12),
Bestimmen eines ersten Wendepunkts (14) der Temperkurve (12),
Bestimmen mindestens eines weiteren zweiten Wendepunkts (15) der Temperkurve (12),
Berechnen der Fläche (12a) oberhalb der Temperkurve (12), begrenzt durch:
die Temperkurve (12), eine horizontale obere Linie (17) durch den Startpunkt (13) und eine vertikale Linie (18) durch den zweiten Punkt (15), oder
Berechnen der Fläche (20) unterhalb der Temperkurve (12), wie begrenzt durch:
eine vertikale Linie (21) durch den Startpunkt (13) und eine horizontale untere Linie (22) durch den zweiten Punkt (15).

7. Computerprogramm nach Anspruch 5 oder **6,** umfassend die Anweisungen:
Bestimmen eines Startpunkts (13) einer maximalen Temperatur der Temperkurve (12),
Bestimmen eines ersten Wendepunkts (14) der Temperkurve (12),
Bestimmen eines zweiten (15) und eines dritten (16) Wendepunkts der Temperkurve (12),
Berechnen der Fläche oberhalb (12a+) der Temperkurve (12), begrenzt durch:
die Temperkurve (12), eine horizontale obere Linie (17) durch den Startpunkt (13) und eine vertikale Linie (19) durch den dritten Punkt (16), oder
Berechnen der Fläche (20) unterhalb der Temperkurve (12), begrenzt durch:
eine vertikale Linie (21) durch den Startpunkt (13) und
eine horizontale untere Linie (22) durch den dritten Punkt (16).

8. Computerprogramm nach einem oder mehreren der Ansprüche 5-7, umfassend die Anweisungen, womit die berechnete Größe der Fläche oberhalb (12a, 12a+) oder unterhalb (20) der Temperkurve (12) durch einen Faktor im Bereich von 1-1000, vorteilhaft 1-100, dividiert wird, wodurch sie in eine tatsächliche Energiemenge konvertiert wird, die zum Kühlen und Verfestigen der jeweiligen Probe erforderlich ist.

## Revendications

1. Procédé destiné à tester et soumettre à un tempérage continu une pâte de confiserie liquide cristallisable contenant des graisses,
dans lequel un échantillon (6) de la pâte est extrait de la pâte liquide avant d'être tempéré, puis refroidi, cristallisé et solidifié, ce qui fournit simultanément une courbe de tempérage (9, 10, 12, 33),
**caractérisé en ce qu'**au moins une partie de la taille de l'aire au-dessus (9a, 10a, 11a, 12a, 12a+, 37) ou en dessous (9b, 20) de la courbe de tempérage (9, 10, 11, 12, 33) est calculée, et **en ce que** la taille réelle de l'aire calculée est appliquée comme représentant la quantité d'énergie réelle nécessaire pour refroidir, cristalliser et solidifier l'échantillon de pâte particulier (6), **en ce que** la taille réelle de l'aire calculée est appliquée dans une autre production d'articles en pâte comme représentant la quantité d'énergie réelle nécessaire pour refroidir, cristalliser et solidifier la pâte particulière,
et **en ce que**, pendant le tempérage continu et la solidification de la pâte sur une ligne de production (23), des échantillons sont prélevés en continu de la pâte tempérée lorsque la taille réelle de l'aire au-dessus (37) ou en dessous de la courbe de tempérage (33) est calculée en continu par un ordinateur et le processus de tempérage est régulé de telle sorte que la taille de l'aire est maintenue à un niveau souhaité, constant.

2. Procédé selon la revendication 2, **caractérisé en ce qu'**on fait baisser la température d'eau de refroidissement pour la pâte en train d'être tempérée quand la taille réelle de l'aire (37) est augmentée, et on fait monter la température d'eau de refroidissement pour la pâte en train d'être tempérée quand la taille réelle de l'aire (37) est diminuée.

3. Ligne de production (23) comportant des moyens pour contrôler le tempérage continu d'une pâte de confiserie cristallisable contenant des graisses et exécuter les étapes d'une ou plusieurs des revendications 1 et 2, laquelle ligne de production (23) comprend au moins un appareil de tempérage (24), lequel appareil de tempérage (24) comprend un tempermètre en ligne (25) avec une unité disposée au niveau d'une conduite d'écoulement continu de pâte (27), laquelle unité est adaptée pour fournir un échantillon de la pâte, comportant en outre une unité de refroidissement (35) adaptée pour refroidir l'échantillon de pâte, et une unité informatique (26) connectée (32) au tempermètre en ligne et adaptée pour calculer et fournir une courbe de tempérage (33) et calculer la taille de l'aire au-dessus (37) ou en dessous de la courbe de tempérage (33) et également adaptée pour convertir la taille réelle de l'aire en une expression pour la quantité d'énergie réelle nécessaire pour refroidir et solidifier l'échantillon de pâte particulier, et dans laquelle un moyen de contrôle pour le circuit de refroidissement de l'appareil de tempérage (24) est connecté à l'unité informatique (26).

4. Ligne de production selon la revendication 3, comprenant en outre un tunnel de refroidissement (31) pour les articles de confiserie, en ce que le moyen de contrôle pour l'unité de refroidissement du tunnel de refroidissement (31) est connecté (39) à l'unité informatique (26).

5. Programme informatique comprenant des instructions pour conduire l'unité informatique d'une ou plusieurs des revendications 3 et 4 d'exécuter une ou plusieurs des étapes des revendications 1 et 2.

6. Programme informatique selon la revendication 5 comprenant les instructions suivantes :
détermination d'un point de départ (13) d'une température maximale de la courbe de tempérage (12),
détermination d'un premier point (14) d'inflexion de la courbe de tempérage (12),
détermination d'au moins un deuxième point supplémentaire (15) d'inflexion de la courbe de tempérage (12),
calcul de l'aire (12a) au-dessus de la courbe de tempérage (12) limitée par :
la courbe de tempérage (12), une ligne horizontale supérieure (17) passant par le point de départ (13) et
une ligne verticale (18) passant par le deuxième point (15), ou
calcul de l'aire (20) en dessous de la courbe de tempérage (12) telle que limitée par :
une ligne verticale (21) passant par le point de départ (13) et une ligne horizontale inférieure (22) passant par le deuxième point (15).

7. Programme informatique selon la revendication 5 ou 6 comprenant les instructions suivantes :
détermination d'un point de départ (13) d'une température maximale de la courbe de tempérage (12),
détermination d'un premier point (14) d'inflexion de la courbe de tempérage (12),
détermination d'un deuxième (15) et d'un troisième (16) points d'inflexion de la courbe de tempérage (12),
calcul de l'aire au-dessus (12a+) de la courbe de tempérage (12) limitée par :
la courbe de tempérage (12), une ligne horizontale supérieure (17) passant par le point de départ (13) et
une ligne verticale (19) passant par le troisième point (16), ou
calcul de l'aire (20) en dessous de la courbe de tempérage (12) limitée par :
une ligne verticale (21) passant par le point de départ (13) et une ligne horizontale inférieure (22) passant par le troisième point (16).

8. Programme informatique selon une ou plusieurs des revendications 5 à 7 comprenant les instructions pour que la taille calculée de l'aire au-dessus (12a, 12a+) ou en dessous (20) de la courbe de tempérage (12) soit divisée par un facteur dans la plage de 1-1000, avantageusement 1-100, ce qui la convertit ainsi en une quantité d'énergie réelle nécessaire pour refroidir et solidifier l'échantillon particulier.
